# EUROPEAN PATENT SPECIFICATION

(11) **EP 2 348 975 B1**
(45) Date of publication and mention of the grant of the patent: **28.03.2018**
(21) Application number: 09825074.9
(22) Date of filing: 27.10.2009
(51) Int. Cl.: A61B 5/021, A61B 5/02, A61B 5/00

(54) **METHOD OF DETERMINING BLOOD PRESSURE AND AN APPARATUS FOR DETERMINING BLOOD PRESSURE**
VERFAHREN ZUR BLUTDRUCKMESSUNG UND VORRICHTUNG ZUR BLUTDRUCKMESSUNG
PROCÉDÉ DE DÉTERMINATION DE LA TENSION ARTÉRIELLE ET APPAREIL DE DÉTERMINATION DE LA TENSION ARTÉRIELLE

(30) Priority: 04.11.2008 SG 200808179
(43) Date of publication of application: 03.08.2011
(73) Proprietor: Healthstats International Pte Ltd, Singapore 536199 (SG)
(72) Inventor: TING, Choon Meng, Singapore 536199 (SG)
(74) Representative: KATZAROV S.A.
(86) International application number: PCT/SG2009/000395
(87) International publication number: WO 2010/053448

(56) References cited:
- EP-A2- 0 247 880
- WO-A1-93/04625
- WO-A2-02/30277
- JP-A- 2006 102 249
- JP-A- 2006 280 769
- JP-A- 2006 288 501
- SU-A1- 1 692 547
- US-A- 4 423 738
- US-A- 4 936 302
- US-A- 5 617 868
- US-A1- 2003 004 421
- US-A1- 2003 191 410
- US-A1- 2003 191 411
- US-A1- 2008 013 777
- Richard Joseph Gowen: "Blood pressure waveforms", , 1961, pages FP-96, XP002751187, Iowa State University of Science and Technology, Ames, Iowa Retrieved from the Internet: URL:http://lib.dr.iastate.edu/cgi/viewcont ent.cgi?article=2358&context=rtd [retrieved on 2015-11-20]
- TANAKA, S. ET AL.: 'Ambulatory instrument for monitoring indirect beat-to- beat blood pressure in superficial temporal artery using volume-compensation method.' MED. & BIOL. ENG. & COMPUT. vol. 34, 1996, pages 441 - 447, XP000636569
- VON MALTZAHN, W. ET AL.: 'Noninvasive blood pressure measurements on the temporal artery.' PROCEEDINGS OF THE ANNUAL INTERNATIONAL CONFERENCE OF THE ENGINEERING IN MEDICINE AND BIOLOGY SOCIETY vol. 14, 29 October 1992 - 01 November 1992, pages 2425 - 2426, XP000347003

## Description

### FIELD OF THE INVENTION

The invention relates to a method of determining blood pressure and an apparatus for determining blood pressure. The invention is particularly directed to determining blood pressure at the superficial temporal artery.

### BACKGROUND TO THE INVENTION

The following discussion of the background to the invention is intended to facilitate an understanding of the present invention. However, it should be appreciated that the discussion is not an acknowledgment or admission that any of the material referred to was published, known or part of the common general knowledge in any jurisdiction as at the priority date of the application.

Blood pressure is commonly measured at arteries positioned in a patient's arm. Such measurements may be taken by invasive means or non-invasive means. Blood pressure measurements in the arteries of the brain are generally measured using invasive methods to determine the pressure at the Internal Carotid artery (CA), and Middle cerebral artery (MCA). This traditional invasive measurement provides early predicators of stroke and the chance of stroke reoccurrence, however due to the invasive nature of such measurements they are not routine tests. There are risks that blood will form a clot around the tip of the catheter, blocking the artery and making it necessary to operate to reopen the vessel. There is a remote risk of the catheter puncturing the artery causing internal bleeding. It is also possible that the catheter tip will separate material from the inner lining of the artery, causing a block downstream in the blood vessel.

Transcranial cardio Doppler TCCD is another method that provides assessment of blood flow velocities in the major cranial vessels using ultrasound techniques. However, the measurements obtained are of the blood movement through the artery. The Doppler must be correctly positioned to ensure there is movement in the direction of the ultrasound beam. Ambiguity in the Doppler signal known as aliasing can occur. This requires adjustments in the pulse repetition by a skilled operator and the pulse repetition frequency may be further constrained by the range of sample volume. Similarly there is a large amount of adjustment as to the frequency used, such adjustment requires a great amount of skill and experience. The choice of frequency is a compromise between better sensitivity to flow (higher frequencies) and better penetration (lower frequencies). The capability of ultrasound to penetrate bone to permit sampling of flow dynamics in the large intracranial vessels is inversely proportional to skull thickness. Thickening of the bone in stroke-age patients, however, may obviate detection.

Patent document JP 2006 280769 A discloses a blood pressure meter to measure blood pressure at a superficial temporal artery.

Accordingly, it is an object of the present invention to determine the blood pressure of a patient at arteries within brain while ameliorating the problems of current devices and techniques.

### SUMMARY OF THE INVENTION

Throughout this document, unless otherwise indicated to the contrary, the terms "comprising", "consisting of", and the like, are to be construed as non-exhaustive, or in other words, as meaning "including, but not limited to".
An apparatus and method enables a reading of blood pressure of the brain at the superficial temporal artery to give an indication of blood related diseases. The apparatus is non-invasive. A reading of blood pressure of the brain is measured as a continuous beat to beat rate on both the left superficial temporal artery and the right superficial temporal artery simultaneously during the same heart beat. Where the waveform measured from the left temporal artery differs from the wave form measured from the right temporal artery this may be an indication of an impending stroke or an indication that a stroke has recently happened. Further the indices of the wave forms may be used as a clinical indication of other blood related diseases.

A device and a method according to the invention is defined in the independent claims.

### BRIEF DESCRIPTION OF THE DRAWINGS

The invention will now be described, by way of example only, with reference to the accompanying drawings, in which:
Figure 1 is a front view of the apparatus.
Figure 2 is a side view demonstrating the connection of the sensor to the head band.
Figure 3 depicts the intended location of use of the apparatus.
Figure 4 depicts the internal location of the superficial temporal artery within the brain.
Figure 5 is a system diagram of the cranial artery network in relation to each other.
Figure 6, 7 and 8 are graphic results of measurement of both the left superficial temporal artery and the right superficial temporal artery in a subject.

### PREFERRED EMBODIMENTS OF THE INVENTION

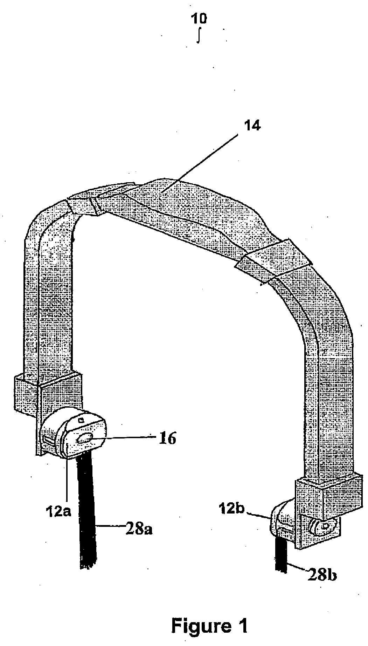

In accordance with a first embodiment of the invention referring to figure 1 there is an apparatus 10 for determining blood pressure. The apparatus 10 comprises a pair of pressure sensors 12. Bridging the two pressure sensors 12 is a head band 14. The head band 14 is curved so that the two pressure sensors 12 are tensioned towards each other so that when it is placed on a wearer the tension of the head band 14 can hold the pressure sensors 12 in place.
Components of the sensor 12 according to the preferred embodiment are similar to those discussed in patent application WO/2002/030277. The sensor 12 according to the preferred embodiment includes a transducer, which produces a voltage output according to pressure changes acting on its diaphragm. A plunger 16 is affixed next to the diaphragm of the transducer. The plunger 16 and sensor 12 of the current system is build to a smaller scale that that disclosed in WO/2002/030277 to negotiate the constraints of the proximity of the superficial temporal artery to the pinna of the ear. It is important that the plunger 16 is able to be positioned such that applanation pressure can be exerted on the superficial temporal artery to allow measurement of a waveform.

The plunger 16 is a specially designed hemispherical component made of a rigid material. In the preferred embodiment the plunger 16 is made of a molded polymer plastic which is biocompatible. The hemispherical surface of the plunger 16 is adapted to push onto the superficial temporal artery of a subject and partially occludes the superficial temporal artery. The base of the plunger 16 housed within the sensor covers the surface of the diaphragm and is in direct contact with the diaphragm.

There is a layer of gel between the diaphragm and the plunger 16 to filter out interference and sharp changes due to unnatural movement. It also dampens the noise ratio. The plunger depth is specially designed such that on most normal heads, it could occlude not more than half the diameter of the superficial temporal artery 102 when the head band 14 is comfortably worn. This will enable full and faithful transmission of the arterial pulsation to be picked up, including the expansion of the arterial walls, the turbulence of the flow and the vibration transmitted along the artery wall from the heart.

As the plunger 16 and the diaphragm are the only moving units at each pulsation, the arterial pressure is accurately picked up as a waveform as each heart beat reaches the superficial temporal artery. For a change in pressure between 0 mmHg-300 mmHg, the displacement of the diaphragm against the pressure variation forms a linear relationship. The range of voltage change in the sensor for such an equation is between 0.5V to 4V, after amplification of the signal. The hemispherical plunger 16 allows for faithful transmission of a continuous beat to bead measurement of the wave form of each heart beat.

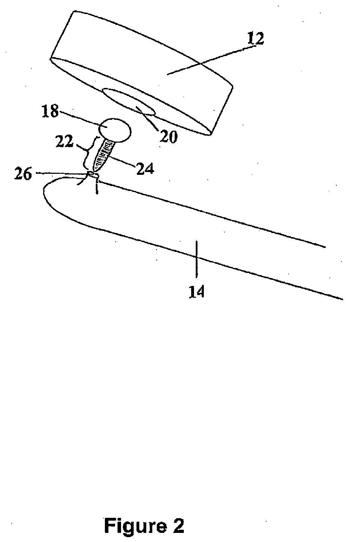

Referring to figure 2 each pressure sensor 12 is attached to one end of the head band 14 at the side opposite the plunger 16. The method of attachment takes the form of a ball joint 18. A bracket 20 is housed within the pressure sensor 12 at the side opposite the plunger 16. A ball joint 18 connected to a linking piece 22 is snapped into the bracket 20 allowing the pressure sensor to be moved to a wide range of locations independent of the location of the head band 14. This structure allows the plunger 16 to be accurately located over the superficial temporal artery of a wide range of wearers. Preferably, the ball joint comprises a spherical or hemispherical head that has a diameter large enough to allow the ball joint to have a large range of movement to negotiate the facial features or facial structures of a subject while not being larger than the width of the end of the head band 14.

Preferably, the linking piece 22 is cylindrical with fine threads 24 that can be screwed into an aperture 26 within one end of the head band 14. The aperture contains tracts to facilitate fine movement of the linking piece 22 into and out of the aperture 26.

Each pressure sensor 12 also is connected to a data and control cable 28. As shown in Figure 1, it is preferred that the point of connection be such that the cable 28 is substantially parallel to the portion of the head band 14 that it also connects to that pressure sensor 12. The cable 28 is connected to a screen such that two separate waveforms can be displayed either side by side or overlapping one another. The waveform is measured in ADC units.

In this manner, the overall visual impact of the apparatus 10 is similar to that of a pair of conventional headphones. The pressure sensors 12 may have padding for the comfort of the wearer

The apparatus 10 will now be described with reference to its intended use.

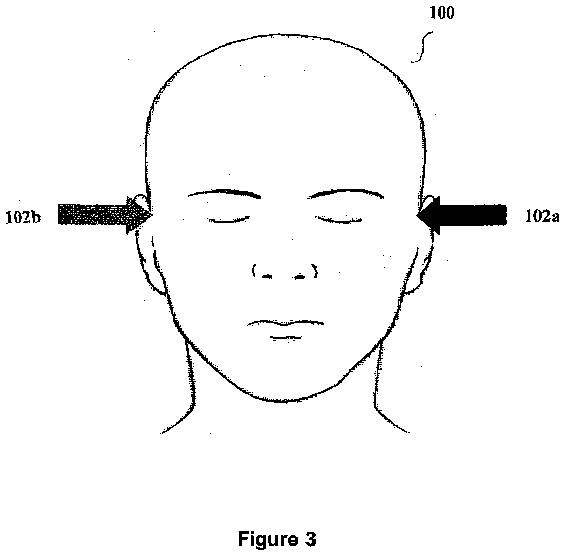

Referring to figure 3, a subject places the apparatus 10 over their head 100. The tensioned curve of the head band 14 ensures that the pressure sensors 12 remain pressed against the side of the head. A medical personnel (not shown) thereafter adjusts the apparatus 10 by swivelling the ball joint 18 within its bracket 20 such that the two plungers 16 are positioned over the superficial temporal artery 102 of the subject 100. This positioning is important as the superficial temporal artery 102 provides the following advantages for blood pressure reading:
- Its position against a bone assists the vertical applanation methodology employed by the pressure sensors 12 to determine blood pressure.
- Commonly, there is very little fat in this region allowing full transmission of the pulse to be recorded as it would not allow for any significant soft tissue compensation.
- The artery is external of the skull bones at this point; and
- The artery is constant in position, as there is no recorded variation in the superficial temporal artery position in humans.

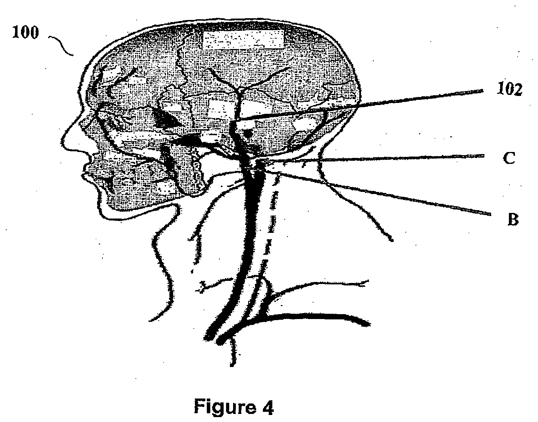

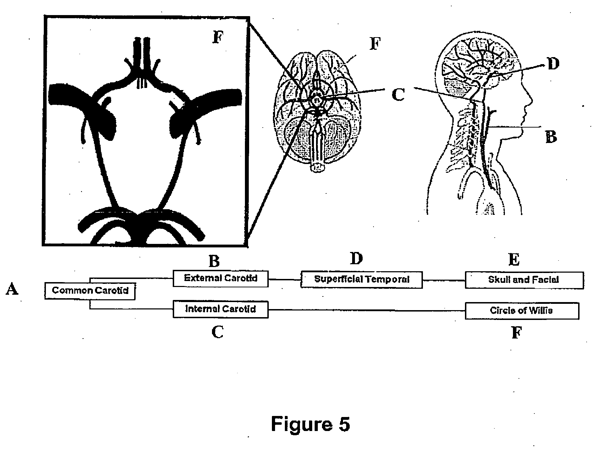

The internal location of the superficial temporal artery is depicted in figure 4 and the system of the cranial artery network in relation to each other is depicted in figure 5. It can be understood that a plague in one of the arteries will affect the blood flow of the interconnected arteries. The location of any obstruction or narrowing will change the blood flow differently depending on the obstructions location and it should be possible to derive a range of indices from a waveform to determine roughly where in the system a problem exists non-invasively.

Following appropriate positioning of the apparatus 10, each sensor 12a, 12b sends its blood pressure measurement signals to a processing station 104 via their respective cables 28a, 28b.

On receipt of the various signals, the processing station 104 separates the measurement data according to the sensor 12 that produced the data.

In the final adjustment the output for both the left superficial temporal artery 102a and the right superficial temporal artery 102b in ADC units is zeroed or levelled. This is achieved by adjusting the fine tuning of the plunger against the superficial temporal artery 102 by turning the linking piece 22 into and out of the aperture 26 at the end of the head band 14. It is important that the applanation pressure on the artery is equalised between both the left superficial temporal artery and the right superficial temporal artery to ensure that any variations between the waveforms measured on both sides is not due to a variation in the applanation pressure. Once the device is secured in position the measurement is quite constant and can be taken over a long time with the wearer being in almost any position.

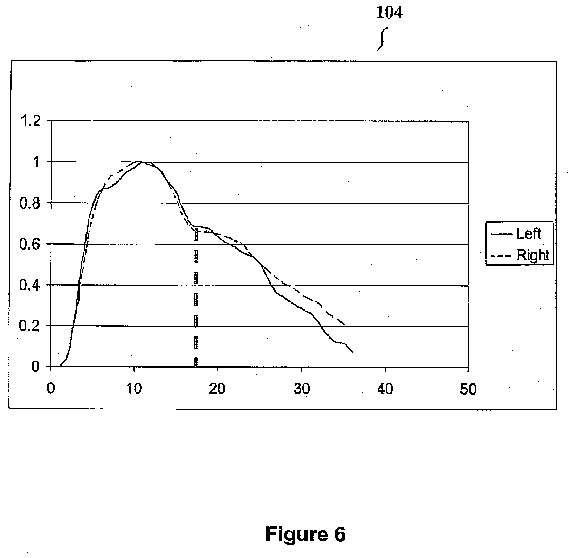

Figure 6, is a display from the processing station 104 showing a measurement of both the left superficial temporal artery and the right superficial temporal artery in a normal subject. While there are very minor variations between the measurements taken from either side the waveforms more or less mirror each other.

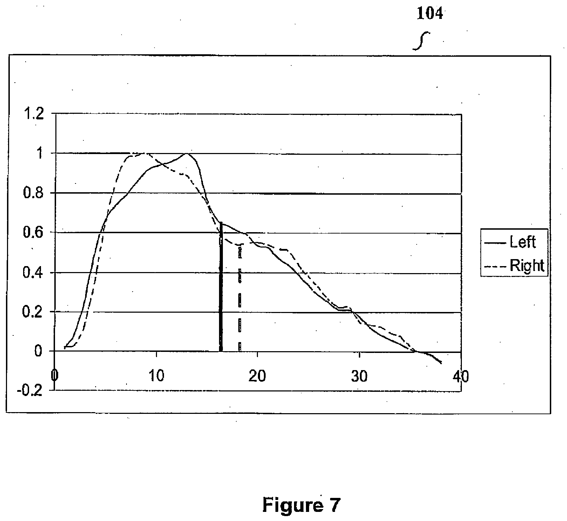

Figure 7 is a display from the processing station 104 showing a measurement of both the left superficial temporal artery and the right superficial temporal artery in a subject that has recently undergone a stroke. The waveforms vary greatly between the left and right side in this case.

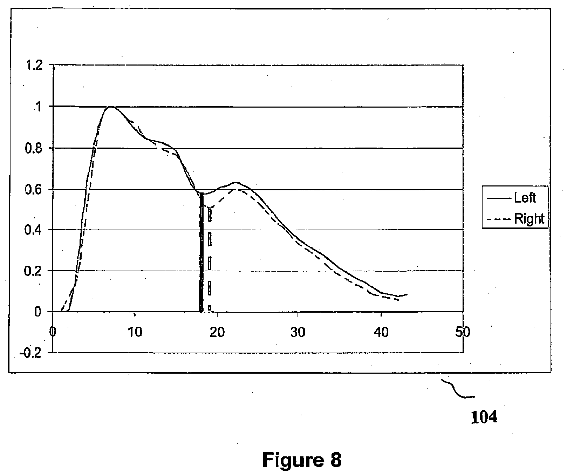

Figure 8 is a display from the processing station 104 showing a measurement of both the left superficial temporal artery and the right superficial temporal artery in a subject who appeared to be normal and had no other indications of a possible impending stroke. There is, however, a variation in the timing of the dicrotic notch which is also higher on the left side than the right side. This subject may be at potential risk of stroke or other such blood related diseases in the brain.

The main aortic artery branches into the left and right common carotid artery A these arteries branch further into two internal carotid arteries C and two external carotid arteries B. The left common carotid artery A branches into the left internal carotid artery C and the left external carotid artery B. Similarly the right common carotid artery A branches into the right internal carotid artery C and the right external carotid artery B. Due to this branching any narrowing or blockages of the arteries will affect blood flow which will be reflected in the waveform measured at the superficial temporal artery. Medical procedures are often limited to arteries in one side of the brain. Similarly problems related to narrowing of the arteries, blockages or other complications may be in one branch eg: in the left internal carotid artery. Measuring and comparing both sides of the main cranial arteries can be used to assess the adequacy of cerebral circulation and blood pressure in the arteries from both sides of the brain.

For example in neonates with extracorporeal membrane oxygenation there is sometimes a need for right common carotid reconstruction that would benefit from monitoring blood pressure in both right and left side arteries. Similarly in high risk stroke cases differences between the blood pressure in the right and the left side arteries using the apparatus 10 provides a non invasive method of detecting obstructive lesions of the arteries or aneurisms that generally only occur in arteries on one rather than both sides of the brain. Such monitoring can be beneficial in people who have never had a stroke or in monitoring post stroke patients. As the measurement is non-invasive which is less risky than invasive methods, the apparatus 10 can be used to monitor a much larger group.

Blood pressure monitoring with the apparatus 10 can be conducted over a long time frame while the person carries out their normal daily activities and recorded so that the data can be passed to a medical practitioner for further analysis.

It should be appreciated by the person skilled in the art that the above invention is not limited to the embodiment described. In particular, the following modifications and improvements may be made without departing from the scope of the present invention:
- The head band 14 may be padded to provide extra comfort to the wearer during use.
- The portion of the head band 14 to which the pressure sensors 12 are connected may be extendible from the remainder of the head band 12. In this manner, the apparatus 10 can be used by people having a wide variety of head sizes and shapes.
- As the use of the ball joint 16 allows free movement of the pressure sensor 12, it is important that the point of connection between the pressure sensor 12 and the data and control cable 22 be at a position such that the weight of the data and control cable 22 does not cause the pressure sensor 12 to move.
- The pressure sensors may be positioned at the superficial temporal artery by a clip or some other attachment means that does not require the headband in which case the sensors may not be connected in any way and may be connected to two separate measuring devices.

## Claims

1. A non-invasive apparatus (10) for measuring blood pressure of the brain at a left superficial temporal artery (102a) and a right superficial temporal artery (102b) of a subject comprising:
a first pressure sensor (12a) and a first adjustment means (18) attached to the first pressure sensor,
a second pressure sensor (12b) and a second adjustment means (18) attached to the second pressure sensor, and
a head band (14) tensioned to allow the first and second pressure sensors to be pressed against the side of a head (100) on the superficial temporal artery when in use each pressure sensor comprising a plunger (16) affixed adjacent a diaphragm of a transducer,
wherein the plunger is a rigid hemispherical component, whereby the first and second adjustment means allows the first and second pressure sensor to be located over the left and right superficial temporal artery of the subject, and the plungers to push into and provide a continuous applanation pressure on the left and right superficial temporal artery of the subject, wherein each plunger comprises a depth that allows the plunger to partially occlude not more than half the diameter of the left and right superficial temporal artery, for continuous beat to beat measurement of a waveform when in use.

2. The non-invasive apparatus of claim 1 wherein the adjustment means comprises a ball joint attached to the pressure sensor via a bracket (20) housed within the pressure sensor whereby the pressure sensor is capable of being moved to a wide range of locations.

3. The non-invasive apparatus of claim 2 wherein the adjustment means further comprises a linking piece (22) connected to the ball joint capable of being screwed into an aperture (26) formed within a head band to facilitate fine adjustment of the pressure sensor on the superficial temporal artery when in use.

4. The non-invasive apparatus of claim 2 whereby the head band is curved to bridge the first pressure sensor and the second pressure sensor so that the two pressure sensors are tensioned towards each other whereby the tension of the head band can hold the first and second pressure sensor on the superficial temporal arteries when in use.

5. The non-invasive apparatus of any one of the preceding claims, wherein there is a layer of gel between the plunger and the diaphragm.

6. The non-invasive apparatus of any one of the preceding claims wherein the pressure sensor is connected to a cable (28).

7. The non-invasive apparatus of claim 6 wherein the cable connects to a processing station (104) where a waveform measured by the sensor when in use is capable of being displayed on the processing station.

8. A non-invasive method for measuring blood pressure of the brain at a left superficial temporal artery and a right superficial temporal artery simultaneously comprising the steps of:
a. Positioning a pressure sensor over the left superficial temporal artery;
b. tensioning a second pressure sensor over the right superficial temporal artery on the head of a wearer such that a first sensor is positioned over a left superficial temporal artery and a second sensor is positioned over a right superficial temporal artery of the wearer, wherein each pressure sensor comprises a plunger affixed adjacent a diaphragm of a transducer and wherein the plunger is a rigid hemispherical component;
c. Adjusting the first and second pressure sensor such that the plungers push into and exert a continuous applanation pressure on the left and right superficial temporal artery, wherein each plunger comprises a depth that allows the plunger to partially occlude not more than half the diameter of the left and right superficial temporal artery; and
d. Measuring a waveform as each heart beat reaches the superficial temporal artery.

9. The non-invasive method of claim 8 further comprising the step of equalizing the applanation pressure between the left superficial temporal artery and the right superficial temporal artery.

10. The non-invasive method of claim 8 or claim 9 wherein the waveform measured from the left superficial temporal artery is compared with the waveform measured

## Patentansprüche

1. Nichtinvasive Vorrichtung (10) zum Messen des Blutdrucks des Gehirns an einer linken oberflächlichen Schläfenarterie (102a) und einer rechten oberflächlichen Schläfenarterie (102b) eines Subjekts, umfassend:
einen ersten Drucksensor (12a) und eine erste Einstelleinrichtung (18), die am ersten Drucksensor angebracht ist,
einen zweiten Drucksensor (12b) und eine zweite Einstelleinrichtung (18), die am zweiten Drucksensor angebracht ist, und
ein Kopfband (14), das gespannt ist, damit der erste und zweite Drucksensor gegen die Seite eines Kopfes (100) auf der oberflächlichen Schläfenarterie wenn im Gebrauch gedrückt werden kann,
wobei jeder Drucksensor einen Stössel (16) umfasst, der angrenzend an einer Membran eines Wandlers befestigt ist, worin der Stössel eine steife hemisphärische Komponente ist,
wobei die erste und zweite Einstelleinrichtung ermöglicht, dass der erste und zweite Drucksensor über der linken und rechten oberflächlichen Schläfenarterie eines Subjekts angeordnet ist, und die Stössel hineindrücken und auf der linken und rechten oberflächlichen Schläfenarterie des Subjekts einen durchgehenden Applanationsdruck ausüben, wobei jeder Stössel jeweils eine Tiefe aufweist, welche es dem Stössel erlaubt, nicht mehr als die Hälfte des Durchmessers der linken und rechten oberflächlichen Schläfenarterie teilweise zu verschliessen, für eine durchgehende Schlag-für-Schlag Messung einer Wellenform, wenn im Gebrauch.

2. Nichtinvasive Vorrichtung gemäss Anspruch 1, wobei das Einstellmittel ein Kugelgelenk aufweist, das am Drucksensor über eine Halterung (20) angebracht ist, die im Drucksensor untergebracht ist, wobei der Drucksensor an eine grosse Vielzahl von Orten bewegt werden kann.

3. Nichtinvasive Vorrichtung gemäss Anspruch 2, worin das Einstellmittel zudem ein Verbindungsstück (22) aufweist, das mit dem Kugelgelenk verbunden ist und in eine in einem Kopfband ausgebildete Öffnung (26) eingeschraubt werden kann, um die Feineinstellung des Drucksensors auf der oberflächlichen Schläfenarterie zu erleichtern, wenn im Gebrauch.

4. Nichtinvasive Vorrichtung gemäss Anspruch 2, wobei das Kopfband gebogen ist, um den ersten Drucksensor und den zweiten Drucksensor zu überbrücken, so dass die beiden Drucksensoren gegeneinander gespannt sind, wobei die Spannung des Kopfbandes den ersten und zweiten Drucksensor an den oberflächlichen Schläfenarterien halten kann, wenn im Gebrauch.

5. Nichtinvasive Vorrichtung gemäss irgendeinem der vorhergehenden Ansprüche, worin sich eine Gelschicht zwischen dem Stössel und der Membran befindet.

6. Nichtinvasive Vorrichtung gemäss irgendeinem der vorhergehenden Ansprüche, worin der Drucksensor mit einem Kabel (28) verbunden ist.

7. Nichtinvasive Vorrichtung gemäss Anspruch 6, worin das Kabel mit einer Verarbeitungsstation (104) verbunden ist,
worin eine vom Sensor bei Gebrauch gemessene Wellenform auf der Verarbeitungsstation angezeigt werden kann.

8. Nichtinvasives Verfahren zur gleichzeitigen Messung des Blutdrucks des Gehirns an einer linken oberflächlichen Schläfenarterie und an einer rechten oberflächlichen Schläfenarterie, umfassend die folgenden Schritte:
a. Positionieren eines Drucksensors über der linken oberflächlichen Schläfenarterie;
b. Spannen eines zweiten Drucksensors über der rechten oberflächlichen Schläfenarterie auf dem Kopf eines Trägers, so dass ein erster Sensor über einer linken oberflächlichen Schläfenarterie positioniert ist und ein zweiter Sensor über einer rechten oberflächlichen Schläfenarterie eines Trägers positioniert ist, worin jeder Drucksensor einen Stössel umfasst, der angrenzend an einer Membran eines Wandlers befestigt ist, und worin der Stössel eine steife hemisphärische Komponente ist;
c. Einstellen des ersten und zweiten Drucksensors, so dass die Stössel hineindrücken und auf der linken und rechten oberflächlichen Schläfenarterie einen durchgehenden Applanationsdruck ausüben, wobei jeder Stössel jeweils eine Tiefe aufweist, welche es dem Stössel erlaubt, nicht mehr als die Hälfte des Durchmessers der linken und rechten oberflächlichen Schläfenarterie teilweise zu verschliessen; und
d. Messen einer Wellenform, wenn jeder Herzschlag die oberflächliche Schläfenarterie erreicht.

9. Nichtinvasives Verfahren gemäss Anspruch 8, zudem umfassend den Schritt des Angleichens des Applanationsdrucks zwischen der linken oberflächlichen Schläfenarterie und der rechten oberflächlichen Schläfenarterie.

10. Nichtinvasives Verfahren nach Anspruch 8 oder Anspruch 9, worin die von der linken oberflächlichen Schläfenarterie gemessene Wellenform mit der von der rechten oberflächlichen Schläfenarterie gemessenen Wellenform verglichen wird, um die zerebrale Blutzirkulation und den Druck in den Arterien auf beiden Seiten des Gehirns zu beurteilen.

## Revendications

1. Appareil (10) non invasif pour mesurer la pression artérielle du cerveau sur une artère temporale superficielle gauche (102a) et une artère temporale superficielle droite (102b) d'un sujet, comprenant :
un premier capteur de pression (12a) et un premier moyen d'ajustement (18) fixé au premier capteur de pression,
un deuxième capteur de pression (12b) et un deuxième moyen d'ajustement (18) fixé au deuxième capteur de pression, et
un serre-tête (14) tendu pour permettre aux capteurs de pression d'être pressés contre le côté d'une tête (100) sur l'artère temporale superficielle lorsqu'en cours d'utilisation,
chaque capteur de pression comprenant un poussoir (16) fixée de manière adjacente à un diaphragme d'un transducteur, le poussoir étant un composant hémisphérique rigide,
dans lequel le premier et deuxième moyen d'ajustement permet au premier et deuxième capteur de pression d'être placé sur l'artère temporale superficielle gauche et droite du sujet, dans lequel chaque poussoir comprend une profondeur qui permet au poussoir de partiellement obturer pas plus que la moitié du diamètre de l'artère temporale superficielle gauche et droite, pour une mesure continue battement par battement d'une forme d'onde lorsqu'en cours d'utilisation.

2. Appareil non invasif selon la revendication 1, dans lequel les moyens d'ajustement comprennent une rotule attachée au capteur de pression par l'intermédiaire d'un support (20) logé dans le capteur de pression, le capteur de pression pouvant être déplacé dans une large plage d'emplacements.

3. Appareil non invasif selon la revendication 2, dans lequel les moyens d'ajustement comprennent en outre une pièce de liaison (22) reliée à la rotule susceptible d'être vissée dans une ouverture (26) ménagée dans un serre-tête pour faciliter le réglage fin du capteur de pression sur l'artère temporale superficielle lorsqu'en cours d'utilisation.

4. Appareil non invasif selon la revendication 2, dans lequel le serre-tête est incurvé pour relier le premier capteur de pression et le deuxième capteur de pression afin que les deux capteurs de pression soient tendus vers l'un par l'autre, dans lequel la tension du serre-tête peut maintenir le premier et le deuxième capteur de pression sur les artères temporales superficielles lorsqu'en cours d'utilisation.

5. Appareil non invasif selon l'une quelconque des revendications précédentes, dans lequel il y a une couche de gel entre le poussoir et le diaphragme.

6. Appareil non invasif selon l'une quelconque des revendications précédentes, dans lequel le capteur de pression est connecté à un câble (28).

7. Appareil non invasif selon la revendication 6, dans lequel le câble se connecte à une station de traitement (104) où une forme d'onde mesurée par le capteur lorsqu'il est en cours d'utilisation est susceptible d'être affichée sur la station de traitement.

8. Procédé non invasif pour mesurer la tension artérielle du cerveau au niveau d'une artère temporale superficielle gauche et d'une artère temporale superficielle droite simultanément, comprenant les étapes suivantes:
a. positionner un capteur de pression sur l'artère temporale superficielle gauche;
b. serrer un deuxième capteur de pression sur l'artère temporale superficielle droite sur la tête d'un porter de telle sorte que le premier capteur est positionné sur l'artère temporale superficielle gauche et un deuxième capteur est positionné sur une artère temporale superficielle droite du porteur, chaque capteur de pression comprenant un poussoir fixé de manière adjacente à un diaphragme d'un transducteur, le poussoir étant un composant hémisphérique rigide ;
c. ajuster le premier et le deuxième capteur de pression de telle manière que les poussoirs poussent dans, et exercent une pression d'aplanation continue sur, l'artère temporale superficielle gauche et droite, chaque poussoir comprenant une profondeur qui permet au poussoir de partiellement obturer pas plus que la moitié du diamètre de l'artère temporale superficielle gauche et droite ; et
d. mesurer une forme d'onde lorsque chaque battement de coeur atteint l'artère temporale superficielle.

9. Procédé non invasif selon la revendication 8, comprenant en outre l'étape d'égalisation de la pression d'aplanation entre l'artère temporale superficielle gauche et l'artère temporale superficielle droite.

10. Procédé non invasif selon la revendication 8 ou 9, dans lequel la forme d'onde mesurée à partir de l'artère temporale superficielle gauche est comparée à la forme d'onde mesurée de l'artère temporale superficielle droite pour évaluer la circulation sanguine cérébrale et la pression artérielle des deux côtés du cerveau.
